# EUROPEAN PATENT APPLICATION

(11) **EP 2 990 052 A1**
(43) Date of publication of application: **02.03.2016**
(21) Application number: 15177622.6
(22) Date of filing: 22.03.2010
(51) Int. Cl.: A61K 38/17, A61K 39/395, A61P 35/00, A61P 37/00

(54) **PHARMACEUTICAL COMPOSTION FOR ACUTE PHASE PROTEIN TARGETED TREATMENT OF ACUTE AND CHRONIC DYSREGULATED INFLAMMATORY DISEASES OR CONDITIONS**

(62) Divisional of application: 10002990.9
(71) Applicant: Linke, Reinhold Paul, 81475 München (DE)
(72) Inventor: Linke, Reinhold Paul, 81475 München (DE); Sander, Leif Erik, New York, 10128-NY (US); Trautwein, Christian, 52066 Aachen (DE)
(74) Representative: Seifert, Ruth

(57) **Abstract**

Disclosed is a Pharmaceutical composition containing as an active ingredient an effective amount of inhibitors of SAA, CRP and/or CXCL-1 activity, or a pharmaceutically acceptable salt for the use in the treatment of dysregulated inflammatory diseases or conditions and/or malignancies and/or cancer and/or tumors by influencing the activity of myeloid-derived suppressor cells (MDSC), as well as the use of an effective amount of inhibitors of SAA, CRP and/or CXCL-1 activity or a pharmaceutically acceptable salt thereof for reducing the frequency, accumulation and/or activity, and/or for depleting MDSC for treating diseases or conditions and a Method for the treatment of malignancies and/or cancer and/or tumors in individuals by administering an effective amount of an inhibitor of SAA, CRP and/or CXCL-1 or a pharmaceutically acceptable salt thereof.

## Description

The present invention describes a novel intervention strategy for the treatment of dysregulated inflammations by using hepatic acute phase proteins serum amyloid A (SAA), CXCL-1/GR0-1/KC or C-reactive protein (CRP) or neutralizing antibodies to these proteins as diseases modulators. The said proteins are to be used as pharmaceutical compositions in the treatment of conditions and diseases characterized by dysregulated inflammations. Besides direct anti-inflammatory as well as host-defensive effects, a hitherto unrecognized regulation of myeloid-derived suppressor cells (MDSC) is uncovered. Myeloid derived suppressor cells seem to be key regulators of inflammatory responses during sepsis and thus represent a valuable target for treatment of these disorders. In a further aspect, the present invention concerns the use of effective amounts of the above identified active agents for activation and mobilisation of MDSC for treatment of excessive dysregulated inflammatory diseases. Further the use of neutralizing antibodies or small molecular inhibitors to treat conditions of suppressed inflammatory responses such as cancer is claimed. The present invention proposes the use of blocking antibodies to SAA, CXCL-1/GR0-1/KC and CRP to prevent MDSC activity and thus boost anti-tumor immune responses in cancer.

### BACKGROUND AND STATE OF THE ART

Dysregulated inflammation is a common culprit of various medical conditions ranging from autoinflammatory diseases like IBD or rheumatoid arthritis (RA) to (polytraumatic) injury and sepsis.

### Acute inflammatory syndromes and conditions

Sepsis is a major cause of mortality worldwide characterised by an uncontrolled inflammatory response to infections. Unfortunately, therapeutic strategies targeting pro-inflammatory cytokines have had only very limited success, demonstrating the complex pathogenesis of disorders that are determined by a variety of both pathogens and host factors.

Uncontrolled activation of the immune system can lead to a systemic inflammatory response syndrome (SIRS). SIRS is most frequently caused by infections in the course of sepsis; non-infectious causes of SIRS include trauma, burns, pancreatitis, ischemia and haemorrhage. Common complications of SIRS include failure of one or more organs or organ systems, e.g. acute kidney injury, acute lung injury like acute respiratory distress syndrome (ARDS), or multiple organ dysfunction syndrome (MODS).

A major complication of sepsis and SIRS is the development of septic shock and subsequent failure of multiple organ systems (MODS) or multiple organ failure syndrome (MOFS) and it is associated with a poor outcome and high mortality. In fact, sepsis and its complications represent the main cause of mortality of intensive care patients. Mortality rate of 20% of patients with severe sepsis and 46% of patients with septic shock has been reported.

A definition of SIRS, sepsis and organ failure etc. as well as guidelines for the use of innovative therapies in sepsis are outlined in Bone, R.C. et.al., Chest 1992, 101, 1644-1655, which is incorporated here by reference. Further, this document provides guidelines for determining sepsis etc. based on different parameters like temperature, heart rate etc.

Treatment of sepsis and SIRS is mainly based on administration of antibiotics, fluid replacement, surgical drainage of infective fluid collections, and appropriate support for organ dysfunction. However, dysregulated inflammatory responses rather than the infectious agent or the trauma itself is often the cause of severe complications that are not sufficiently targeted by the general therapeutic strategies outlined above. Thus, significant efforts have been made to inhibit key mediators of inflammation, yet most studies yielded rather discouraging results. Hence, there is an urgent need for alternative anti-inflammatory strategies.

### Chronic inflammatory conditions

Diseases characterized by chronic inflammation include autoinflammatory and autoimmune diseases like inflammatory bowel disease (IBD), RA (rheumatoid arthritis) or psoriasis. Here chronic activation of immune cells drives tissue destruction, which can proceed to a loss of organ functions and severe complications such as intestinal strictures, joint destructions and malformations. In addition chronic inflammatory organ damage is considered a major factor contributing to cancer development and liver cirrhosis.

### Acute Phase Proteins

Acute phase proteins (APPs) are an evolutionarily conserved family of proteins produced mainly in the liver in response to infections and inflammations. They are defined as proteins whose serum levels change by more than 25 % during inflammation. Some APPs increase (positive APPs) and others decrease (negative APPs) The positive APPs (in the following referred to as APPs) are mainly synthesized in the liver and are regarded as important components of the innate immune response to infection. Many APPs are known as potent opsonins and activators of innate immune cells like neutrophils, but they also have some anti-inflammatory properties. Due to the large diversity of APPs with pro- and anti-inflammatory function their overall role during infections is still not well defined.

Interleukin-6 (IL-6) is one of the major inducer of APP production in hepatocytes in vitro. IL-6 shares the common signaling receptor gp130 with eight other cytokines. Innate immune cell activation is critical for host defence against invading microorganisms and for the subsequent generation of an adaptive immune response. On the other hand, pro-inflammatory mediators produced by innate immune cells are considered key elements in the pathogenesis of severe sepsis and multi-organ failure. A complex network of activating and inhibiting functions leading to regulatory pathways controls innate immune responses. It is known that IL-6 represents also one of the major pro-inflammatory mediators and of sepsis is a strong stimulator of APP production in the liver in vivo. Notably, patients with chronic liver diseases have a significantly increased risk of acquiring sepsis and its accompanying complications, and, thus, have higher sepsis related mortality. Septic patients with chronic liver disease exhibit prolonged exacerbated inflammation and elevated levels of inflammatory cytokines.

As mentioned above, APPs form a large group of proteins with very diverse functions. APPs include molecules such as CRP (C-reactive protein) and SAA (serum amyloid A).

CRP is a commonly used clinical acute phase response and APP-marker in patients. Its serum levels have been shown to correlate well with systemic or local inflammatory responses. CRP has been shown to bind phosphocholine residues expressed on the surface of dead or dying cells as well as on some bacteria, which results in the activation of the complement system. Complement activation constitutes a major innate defense mechanisms against bacterial invaders. CRP is produced in the liver by hepatocytes in response to gp130- activation by IL-6 or other IL-6 family cytokines.

Serum amyloid A protein (SAA) constitutes a highly conserved multi-gene apolipoprotein family consisting of four genes in humans of which only three are expressed with SAA3 being a pseudogene. SAA 1 and SAA2 comprise the acute phase SAAs (A-SAA) which are chemically closely related with less than 10 % different of the amino acids sequence while SAA4 differs by 47% and 45% from SAA 1 and SAA2, respectively. In addition, A-SAA-allotypes (SAA 1.1-5 and SAA2.1-2) are known. Since SAA4 does not show the conspicuous acute phase behaviour it is called constitutive SAA (C-SAA) and its function is largely unknown. The A-SAAs have 104 amino acids while C-SAA has 112 amino acids (NM 199161). The larger size of the latter is due to an insertion of an octapeptide between residue 69 and 70 whose function is unknown. All SAA molecules in all species analyzed so far share an invariant peptide at position 33-43 which has been shown to convey major SAA-functions by being blocked through monoclonal antibodies directed against this peptide. In addition within this invariant peptide the tripeptide RGN has been noticed. It resembles the RGD cell-binding motive of fibronectin which is one of the cell adhesive proteins within the extracellular matrix and which can also permit neoplastic cell to home and build metastases in the connective tissue displaying these cell binding motives In addition, the peptide YIGSD (Seq. ID. No. 1) at position 29-33 only on SAA1 resembles the cell adhesive motive YIGSR (Seq. ID. No. 2) of the extracellular matrix protein laminin. Both motives on SAA can counteract the homing of metastatic cells by competing with similar motives on fibrinectin and laminin (as examples of families of cells having similar functions) in the various connective tissues. SAA can also become pathogenic after various long lasting acute phase reactions by the polymerization to amyloid of the SAA 1/2 fragment1-76 which causes the fatal disease AA amyloidosis.

SAA has been associated with a number of chronic and acute inflammatory conditions regardless of origin. In response to acute inflammatory stimuli, which are in particular represented by the monocyte-derived IL-1, IL6 and TNFα, plasma levels of SAA1 and SAA2 can rapidly increase more than 1,000 fold within 6 to 12 hours.

CXCL1 (NM 001511, Seq ID. No.3) is a small cytokine belonging to the Cxc chemokine family. CXCL1 plays a role in spinal cord development and is involved in the processes of angiogenesis, inflammation, wound healing and tumorigenesis. It is mainly known for its role in recruiting polymorphonuclear leukocytes to the site of inflammation through its direct chemotactic activity. CXCL1 exerts its functions through the chemokine receptor CXCR2.

Given the large number and vast diversity of APPs with both pro- and anti-inflammatory functions, their overall role during dysregulated inflammatory diseases e.g. sepsis, remains still ill defined.

### Myeloid Derived Suppressor Cells (MDSC)

Myeloid derived suppressor cells (MDSC) represent a heterogeneous, immature population of myeloid (precursor) cells that are commonly characterised by the expression of the myeloid lineage markers Gr1 and CD11b in mice and are known to inhibit T effector responses. Myeloid cells with anti-inflammatory functions have been recognized in tumor-bearing mice for a long time (Young et al., 1987), but their functional significance for tumor immune evasion, angiogenesis and metastasis has only recently been more broadly appreciated (Nagaraj et al., 2007; Yang et al., 2008). MDSC have also been implicated in other pathological conditions like autoimmunity. Expansion of MDSC with T cell suppressive properties during sepsis in mice has been reported (Delano et al., 2007). Unlike for many cancers, their contribution to disease progression and host survival during infections is still undefined.

Interestingly, MDSC have also been shown to play a protective role in models of autoinflammatory diseases like IBD (Haile et al., 2008). Importantly their presence has also been demonstrated in humans (Hoechst et al., 2008).

As mentioned above, MDSC have pro-tumorigenic functions and inhibition of MDSC is considered a promising future therapeutic strategy (Gabrilovich andNagaraj, 2009). In humans MDSCs have been characterized by various surface markers including CD34, CD33, CD15, and CD13, CD14-/lin-, or CD11b, CD66, VEGFR.

There is an unmet need for therapeutic agents to treat dysregulated inflammatory diseases or conditions, in particular sepsis and SIRS. In addition, immune evasion by cancers represents a major problem, which could be targeted through the same mechanisms that control inflammation during sepsis and SIRS.

### SUMMARY OF THE PRESENT INVENTION

Using a liver specific gp130-knock out mouse, it is demonstrated that hepatocyte derived SAA and CXCL1/KC are crucial regulators of innate inflammation during polymicrobial sepsis in mice. Employing complementary loss and gain of function experiments clear evidence for the protective role of SAA during sepsis, by controlling inflammation and promoting survival, without affecting bacterial clearance as provided. Thus a major mechanism of protection mediated by liver derived SAA and KC as described in greater detail in the relevant section below has been delineated.

Further, it is discovered that the chemokine CXCL1/GRO1 (KC in mice) is strongly upregulated in hepatocytes in a gp130-dependent manner during polymicrobial sepsis in mice, which correlates directly with its serum levels.

The present invention provides a novel therapeutic strategy for the treatment of dysregulated inflammation, in particular acute uncontrolled inflammation during sepsis, severe sepsis, septic shock and SIRS. The current invention proposes the use of recombinant proteins or their biologically active motifs in effective amounts of SAA or CXCL1/GRO1 to control dysregulated inflammation and prevent subsequent organ damage or death. Administration of these compounds actively promotes the function of MDSC.

That is, the present invention provides a pharmaceutical composition containing as an active ingredient an effective amount of SAA, CRP and/or CXCL1 or analogs or active fragments thereof or ii) inhibitors of SAA, CRP and/or CXCL activity or a pharmaceutical salt thereof for the use in the treatment of dysregulated inflammatory diseases or conditions. Said dysregulated inflammatory diseases or conditions are treated by influencing the peripheral frequency, accumulation and activity of myeloid-derived suppressor cells (MDSC).

Preferably, said dysregulated inflammatory diseases or conditions are SIRS, sepsis, severe sepsis, septic shock, multi organ dysfunction syndrome (MODS), MOFS, or acute transplant rejection.

It is also preferred that the pharmaceutical composition contains as an active ingredient inhibitors of SAA, CRP and/or CXCL1 activity for use in the treatment of malignancies of dysregulated inflammatory diseases or conditions whereby the inflammation response is suppressed, through inhibition of MDSC accumulation, frequency and/or activity.

In another embodiment, the present invention provides the use of an effective amount of SAA, CRP and/or CXCL1 or analogs or active fragments thereof, or pharmaceutical acceptable salts thereof for activation and mobilisation of MDSC for treating excessive dysregulated inflammatory diseases or conditions. On the other hand, inhibitors of SAA, CRP and/or CXCL1 are useful for producing the frequency, accumulation and/or activity, and/or for depleting MDSC for treating diseases or conditions characterised by suppressed immune response.

Finally, methods for treating dysregulated inflammatory diseases or conditions using the compounds as described herein are contemplated.

### BRIEF DESCRIPTION OF THE FIGURES

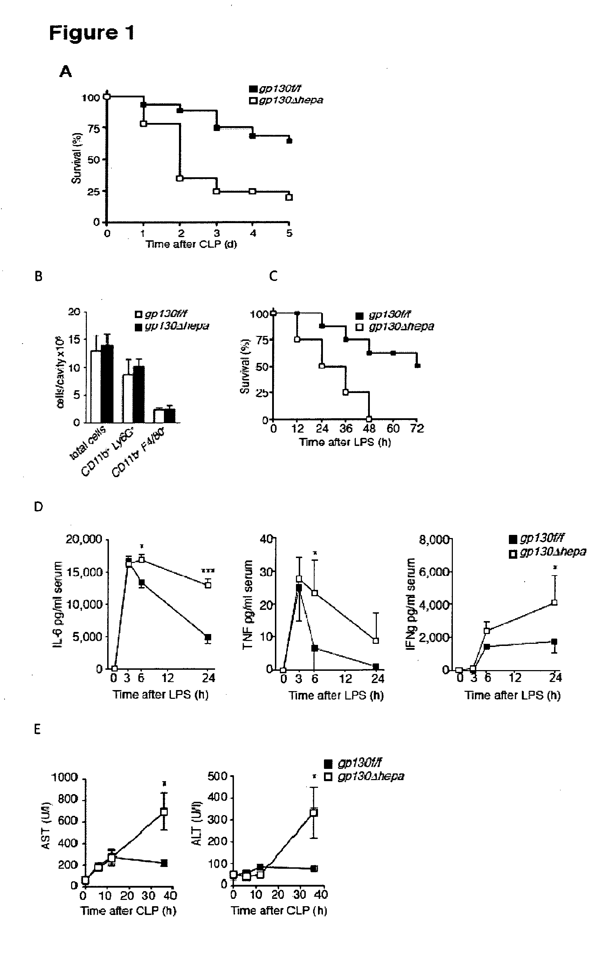

*Fig. 1**. Gp130*/*Stat3 signaling in hepatocytes is protective during polymicrobial sepsis and tempers the inflammatory response.*
   **(A)** Decreased survival of *gp130Δhepa* mice after CLP (n= 45 per group, pooled data of 6 independent experiments). **(B)** Recruitment of immune cells to the peritoneal cavity was equivalent in *gp130Δhepa* and control mice. Peritoneal cavities were lavaged 1Bh after CLP, and infiltrating cells were counted and analyzed by flow cytometry. CD11b⁺Ly6G⁺ neutrophils, CD11b⁺F4/80⁺; monocytes and macrophages (n=5 per group, 1 of 3 independent experiments shown). **(C)** Decreased survival of *gp130Δhepa* mice during endotoxic shock. Mice were treated with 20mg/kg LPS and survival was determined. (n= 10 per group, pooled data of 3 independent experiments). **(D)** Serum cytokine levels in *gp130f*/*f* and *gp130Δhepa* mice after LPS treatment were measured by ELISA (8 per group, pooled data of 3 independent experiments). (E) Increased liver damage in *gp130Δhepa* mice after CLP. Serum levels of AST (left) and ALT (right), (n= 5 per group, 1 out of 3 independent experiments shown) **(J)** Representative H&E staining of histological sections of liver tissue. (*; p<0.05, **; p<0.01, ***; p<0.001, data are presented as mean ±SEM)

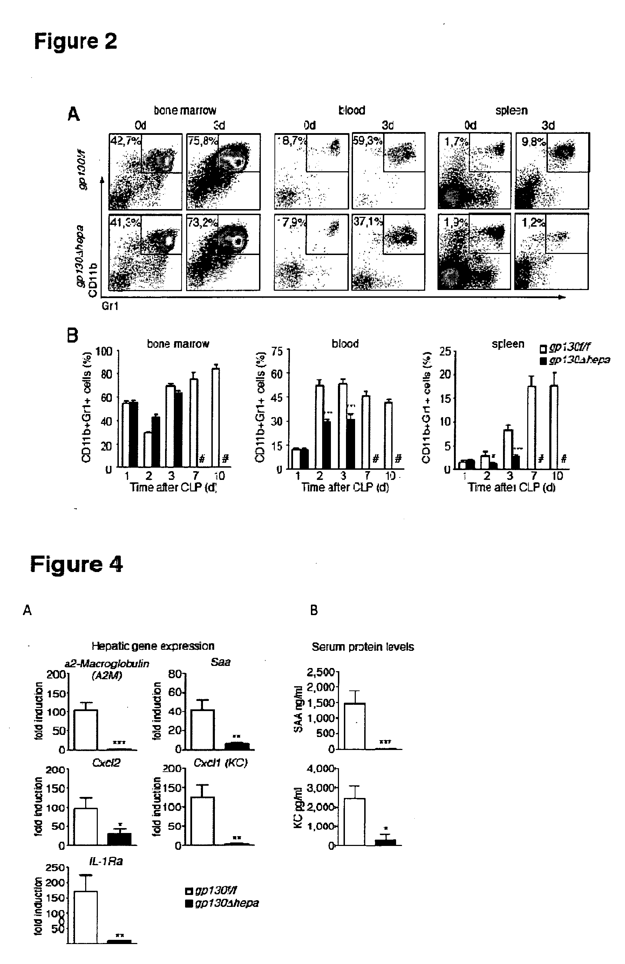

*Fig. 2**. Mobilization and splenic accumulation of CD11b+Gr1* + *MDSC is impaired in gp130Δhepa mice.*
   **(A)** Representative flow cytometric analysis and **(B)** graphic analysis of CD11b⁺Gr1⁺ cell frequencies in bone marrow, peripheral blood and spleens in *gp130f*/*f* and *gp130Δhepa* mice at indicated time points after CLP (n= 5-12 per time point per group, pooled data of 12 independent experiments. #; *gp130f*/*f* didnot survive to 7 and 10 d in sufficient numbers and could therefore not be included in the analysis). (*; p<0.05, **; p<0.01, ***; p<0.001)

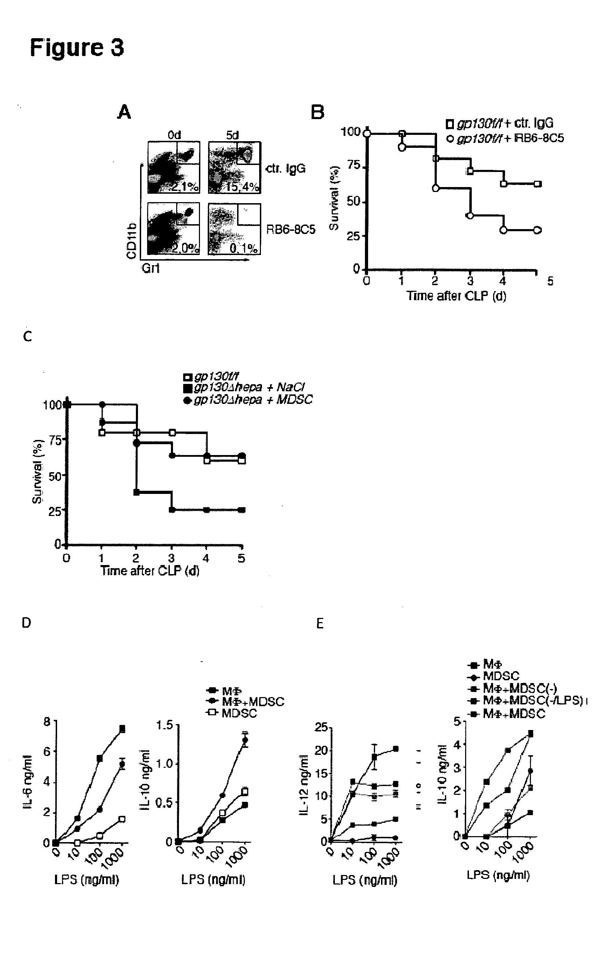

*Fig. 3**. MDSC regulate inflammatory responses and confer protection during sepsis.*
   **(A, B)** Depletion of Gr1 expressing cells decreased survival after CLP. *Gp130f*/*f* mice were injected with monoclonal antibodies against Gr1 (RB6-8C5) 24 h before and after CLP. Efficient depletion of MDSC was determined by flow cytometry of splenocytes (A). Survival was monitored (B, n= 9 per group, pooled data of 3 independent experiments). **(C)** *Gp130Δhepa* mice received either 5x10⁶ MDSC or saline 1 h and 24 h after CLP and survival was determined (n= 12 per group, pooled data of 3 independent experiments). **(D)** MDSC skew LPS induced cytokine production of peritoneal macrophages (MΦ). Macrophages were isolated from untreated *gp130f*/*f* mice and co-cultured with MDSC at a ratio of 1:3 and stimulated with LPS at indicated concentrations. Cytokine release was determined by ELISA (1 of 5 independent experiments shown). **(E)** Gr1⁺CD11b⁺ were isolated from spleens of septic mice (MDSC) or naïve mice (MDSC(-)) and co-cultured with macrophages. Some cells from naïve mice were pre-treated with 100ng/ml LPS in vitro prior to co-culture with macrophages (MDSC(-/LPS)). **(E:** 1 of 3 independent experiments shown). (*; p<0.05, **; p<0.01, ***; p<0.001, data are presented as mean ±SEM)
*Fig. 4**. Hepatocytes launch a specific gp130-dependent transcriptional program in response to infection.*
   **(A)** Quantitative real time PCR of liver samples taken from untreated *gp130f*/*f* and *gp130Δhepa* mice and 12 h after induction of sepsis (n= 8 per group, pooled data of 3 independent experiments). Acute phase proteins (alpha-2- macroglobulin, SAA), chemokines (KC, CxCI-2) and antiinflammatory cytokines(IL-1Ra) were analyzed. **(B)** Serum protein levels of SAA (upper graph) and KC (lower graph) were analyzed by ELISA in *gp130f*/*f* and *gp130Δhepa* mice 24 h after CLP (n= 7 per group, 3 independent experiments). (*; p<0.05, **; p<0.01,***; p<0.001, data are presented as mean ±SEM)

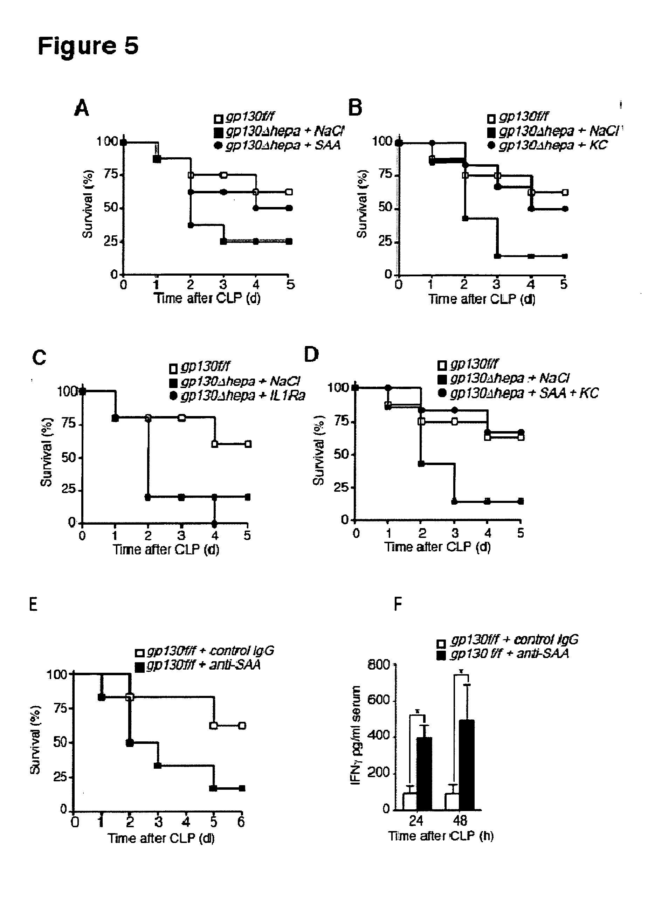

*Fig.* 5. *Recombinant SAA and KC are protective during sepsis.*
   Survival of *gp130f*/*f* mice compared to *gp130Δhepa* mice treated with **(A)** SAA, **(B)** KC, **(C)** IL-1Ra (Anakinra) or **(D)** SAA and KC in combination or equal volumes of NaCl 1 h and 24 h after CLP (n= 9 per group, pooled data of 3 independent experiments). **(E)** Survival of *gp130f*/*f* mice treated with neutralizing anti-SAA antibodies or inactive control isotype IgG. (F) IFN-y serum levels at indicated time points after CLP in *gp130f*/*f* mice treated with anti-SAA or control antibodies (n= 6 per group, pooled data of 2 independent experiments). (*; p<0.05, data are presented as mean ±SEM).

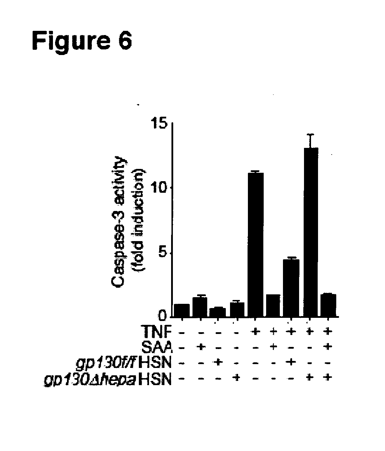

*Fig. 6**. Hepatocyte derived SAA reconstitute splenic MDSC and protects*
   *them from apoptosis.*
   **(A)** *In vitro* apoptosis of MDSC induced by TNF can be inhibited by SAA or supernatants from primary hepatocytes isolated from *gp130f*/*f (gp130f*/*f* HSN) but not from *gp130Δhepa (gp130Δhepa* HSN) mice. Hepatocytes were stimulated with the potent APP inducer and gp130 ligand leukemia inhibitory factor for 6 h. Apoptosis of MDSC was determined by measuring Caspase-3 activity. (1 of 3 independent experiments shown. Data are presented as mean ±SEM).

### DETAILED DESCRIPTION OF THE PRESENT INVENTION

The present inventors found that the hepatic APPs, like SAA, CRP and CXCL1, are critical to control inflammatory responses during dysregulated inflammatory diseases, sepsis, and administration of recombinant APPs represent an efficient treatment of dysregulated inflammatory diseases. They found that these protective effects are mediated at least in parts by promoting the activity of MDSC. The present inventors discovered a hitherto unrecognized role for MDSC in protecting the host against overshooting inflammation e.g. during sepsis, without affecting anti-microbial defence and bacterial clearance. Thus, SAA, CRP and CXCL1 are useful for the treatment of dysregulated inflammatory diseases or conditions. SAA, CRP and CXCL1 (KC) promote MDSC mobilisation, accumulation and survival. Peripheral accumulation of MDSC inhibits excessive inflammatory responses. Therefore, targeting MDSC during sepsis through administration of SAA, CRP and/or CXCL1 represents a novel promising therapeutic option.

In addition, regulating MDSC activity, in particular MDSC mobilisation, accumulation and survival allows treating of systemic inflammatory response syndrome, sepsis, severe sepsis, septic shock and MODS. The present inventors discovered that the gp130 depended communication between the liver and MDSC through acute phase proteins SAA, CRP and CXCL1 critically controls inflammatory responses or conditions, e.g. during infection.

Hence, in a first aspect, the present invention relates to pharmaceutical compositions containing as an active ingredient an effective amount of i) SAA, CRP and/or CXCL-1 or analogs or active fragments thereof or ii) inhibitors of SAA, CRP and/or CXCL-1 activity, or a pharmaceutically acceptable salt of i) or ii) for the use in the treatment of dysregulated inflammatory diseases or conditions by influencing the activity of myeloid-derived suppressor cells (MDSC).

In a preferred embodiment, the pharmaceutical composition contains as an active ingredient an effective amount of SAA, CRP and/or CXCL-1 or analogs or active fragments thereof, or a pharmaceutically acceptable salt thereof for use in the reduction, amelioration or termination of inflammatory responses or slow down the propagation or further escalation of inflammation or a disease associated with dysregulated inflammatory diseases or conditions by enhancing the activity of MDSC.

Moreover, another aspect of the present invention, a pharmaceutical composition is provided for use in the treatment of autoinflammatory diseases or autoinflammatory diseases with autoimmune components or autoimmune diseases including IBD, rheumatoid arthritis, psoriasis, multiple sclerosis, autoimmune hepatitis, transplant rejection, graft vs. host and host vs. graft disease, acute toxic and chronic toxic liver damage, local and systemic inflammatory diseases.

In a further aspect of the present invention, a pharmaceutical composition is provided for use in the treatment of dysregulated inflammatory diseases or conditions whereby the inflammation response is suppressed, for the treatment of tumors or cancer by reducing the activity of MDSC, e.g. by inducing apoptosis of MDSC, said pharmaceutical composition comprises as an active ingredient an effective amount of inhibitors of CRP, SAA, and/or CXCL1 activity. Preferably, said inhibitors are neutralising antibodies against SAA, CRP and/or KC or a pharmaceutically acceptable salt thereof.

According to the present invention, the term "serum amyloid A" (SAA) refers to the full length SAA molecules known as SAA1, SAA2, etc. The term also contemplates analogs or functional fragments of SAA, in particular of human SAA of gene bank accession No. NM_000331 (Seq. ID No. 5), and NM_030754 (Seq. ID. No. 7), P02735.

As used herein, the term "C reactive protein" (CRP) refers to the full length protein, e.g. the human protein NM_000567 (Seq. ID. No. 9), P02741. The present invention contemplates analogs as well as functional fragments of C reactive protein.

The term "CXCL1" as used herein refers to the CXCL1 chemokine also known as KC or GR01, having the gene bank acsession number J03561 and NM 001511 (human form), Seq. ID. No. 3, P09341. The present invention contemplates analogs as well as functional fragments of CXCL1.

As used herein, the term "a functional fragment thereof" refers to a fragment of the respective protein, e.g. a peptide which display the same activity. In the present document, the term "SAA", "CRP" and "CXCL1" includes respective fragments thereof unless otherwise indicated. Further unless explicitly noted, said terms includes salts or solvates of the compounds.

The term "analog" as used herein refers to molecules having the same activity as SAA, CRP and CXCL1, respectively. In particular, an analog has the same activity as the respective full length proteins in activating and mobilising MDSC. The term "CRP", "SAA" and "CXCL1" includes respective analogs which may be present in a salt or solvate form unless otherwise indicated.

In a preferred embodiment, the SAA, CRP and CXCL1 molecules are proteins of SEQ ID Nos. 4, 6, 8, 10, respectively.

The active ingredients described herein may be in the form of pharmaceutically acceptable non-toxic salts thereof or other galenic preparations. Salts include acid added salts, such as salts with inorganic acids or with organic acids and also salts formed with cationic ions, like metallic ions, in particular alkali or alkaline health metal 1 or NH4+. Further, the active ingredients may be present in form of solvates thereof (e.g. hydrates).

Inhibitors of SAA, CRP and/or CXCL1 activity refer to compounds able to suppress or inhibit the activity of the respective protein, in particular, its activity to mobilise and activate MDSC and suppress inflammatory responses. Said inhibitors may be small molecules, peptides, polypeptides, proteins, in particular antibodies, including neutralising antibodies and/or Fc fusion proteins. In this context, the term "small molecule" particularly refers to small organic molecules. Furthermore, said inhibitors may be in form of biological molecules, like antibodies. For example, said inhibitors are neutralising antibodies neutralising the activity of SAA, CRP and/or CXCL1, respectively. The inhibitor may act directly on CRP, SAA and/or CXCL1 or may act indirectly by binding to factors that bind to said proteins or nucleic acids encoding the same. Whatever mechanism is involved, as used herein, inhibitors of CRP, SAA and/or CXCL1 inhibit or suppress the activity of the respective proteins. In particular, this neutralizing activity will be exploited to inhibit to mobilisation, accumulation, activation or survival of MDSC.

In addition, the pharmaceutical composition comprising as an active ingredient SAA, CRP and/or KC are useful in the therapy of systemic inflammatory response syndrome, sepsis, severe sepsis, septic shock, multi organ dysfunction syndrome, or acute transplant syndrome.

It is preferred that the pharmaceutical composition comprises at least an effective amount of CRP or an inhibitor of CRP or a pharmaceutically acceptable salt thereof. Moreover, it is preferred that the pharmaceutical composition comprises as an active ingredient an effective amount of SAA or an inhibitor of SAA or a pharmaceutically acceptable salt thereof. In addition, it is preferred that either a combination of effective amounts of SAA and KC or a combination of effective amounts of CRP and KC are present in the pharmaceutical composition according to the present invention.

In preferred embodiments, the pharmaceutical composition further comprises pharmaceutical additives or auxiliary substances, preferably, a pharmaceutically acceptable carrier, excipient or diluent.

The term "pharmaceutical composition" means a composition suitable for pharmaceutical use in a subject or individual, including an animal or human. A pharmaceutical composition generally comprises an effective amount of an active agent or ingredient and a carrier, including e.g. a pharmaceutically acceptable carrier.

A "therapeutic treatment" is a treatment administered to a subject or an individual to display symptoms or signs of pathology, disease, or disorder in which treatment is administered into the subject for the purpose of diminishing or eliminating those signs or symptoms of pathology, disease or disorder.

As used herein, the term "carrier" refers to a diluent, adjuvant, excipient or vehicle.

The composition comprising the compounds according to the present invention will be formulated, dosed, and administered in a fashion consistent with good medical practice. Factors for consideration in this context include the stage of the particular disease or disorder being treated, the particular mammal being treated, the clinical condition of the individual subject, the site of delivery of the agent, the method of administration, the scheduling of administration, and other factors known to medical practitioners. The effective amount of the agent to be administered will be governed by such considerations. The key factor in selecting an appropriate dose and scheduling is the result obtained, as indicated above. The compounds according to the present invention are administered by any suitable means, including parenteral, topical, subcutaneous, intraperitoneal, intrapulmonary, intranasal, and/or intralesional administration. Parenteral infusions include intramuscular, intravenous, intraarterial, intraperitoneal, or subcutaneous administration. Intrathecal administration is also contemplated.

"Therapeutically- or pharmaceutically-effective amount" as applied to the compositions of the instant invention refers to the amount of composition sufficient to induce a desired biological result. That result can be alleviation of the signs and markers, symptoms, or causes of a disease, or any other desired alteration of a biological system. In the present invention, the result will typically influence inflammatory response, including cytokine inflammatory mediator levels as well as frequency and activity of MDSC either by activating or mobilising said cells or by suppressing their activity including apoptosis induction.

In vitro assays as well as animal models may optionally be employed to help identifying optimal dosage ranges. The precise dose to be employed in theformulation will also depend on the route of administration, and the seriousness of the disease or disorder, and should be decided according to the judgment of the practitioner and each patient's circumstances. Effective doses may be extrapolated from dose-response curves derived from in vitro or animal model test systems. Preferably, the pharmaceutical composition is administered directly or in combination with an adjuvant.

The term "administered" means administration of a therapeutically effective dose of the aforementioned pharmaceutical composition comprising the active ingredient according to the present invention in form of salts and solvates thereof to an individual.

The pharmaceutical composition according to the present invention comprises the active ingredient as described herein and, optionally, a pharmaceutical acceptable carrier. Such pharmaceutical compositions comprise a therapeutically effective amount of the active ingredient as defined herein and, optionally, a pharmaceutically acceptable carrier. The pharmaceutical composition may be administered with a physiologically acceptable carrier to a patient, as described herein. Acceptable means that the carrier be acceptable in the sense of being compatible with the other ingredients of the composition and not be deleterious to the recipient thereof. In a specific embodiment, the term "pharmaceutically acceptable" means approved by a regulatory agency or other generally recognized pharmacopoeia for use in animals, and more particularly in humans. The term "carrier" refers to a diluent, adjuvant, excipient, or vehiclewith which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations, aerosols and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium, carbonate, etc. Examples of suitable pharmaceutical carriers are described in "Remington's Pharmaceutical Sciences" by E.W. Martin (18th ed., Mack Publishing Co., Easton, PA (1990)). Such compositions will contain a therapeutically effective amount of the aforementioned compounds, salts or solvates thereof, preferably in purified form, together with a suitable amount of carrier so as to provide the form for proper administration to the patient. The formulation should suit the mode of administration.

Typically, pharmaceutically or therapeutically acceptable carrier is a carrier medium which does not interfere with the effectiveness of the biological activity of the active ingredients and which is not toxic to the host or patient.

In another preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in aunit dosage form, for example, as a dry lyophilised powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The pharmaceutical composition for use according to the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

These and other embodiments are disclosed and encompassed by the description and examples of the present invention. Further literature concerning any one of the methods, uses and compounds to be employed in accordance with the present invention may be retrieved from public libraries, using for example electronic devices.

In a further embodiment, the present invention relates to the use of an effective amount of SAA, CRP and/or CXCL1 or pharmaceutically acceptablesalts thereof for activation and mobilisation as well as for survival of MDSC e.g.for treating excessive dysregulated inflammatory diseases.

That is, when excessive dysregulated inflammatory diseases, like it is the case in sepsis, occurs in an individual, the compounds according to the present invention are administered to said individual afflicted with an excessive dysregulated inflammatory disease to ameliorate to slow down said inflammation, or to reduce escalation of the inflammation or to terminate inflammatory responses.

Moreover, the inventors recognise that the use of an effective amount of inhibitors of CRP-, SAA- and/or CXCL1 activity or pharmaceutically acceptable salts thereof are useful for reducing the activity and/or are useful for deleting MDSC. This will allow treating suppressed dysregulated inflammatory diseasesor conditions.

In another embodiment, the present invention relates to the use of an effective amount of CRP, SAA and/or CXCL1 or pharmaceutically acceptable salts or solvates thereof for suppressing inflammation in individuals afflicted with dyregulated inflammatory diseases or conditions.

Finally, the present invention relates to methods for treating individuals afflicted with dysregulated inflammatory diseases or conditions, like sepsis, comprising the step of administering an effective amount of an active ingredient of CRP, SAA and/or CXCL1 or inhibitors of CRP, SAA and/or CXCL1 or pharmaceutically acceptable salts thereof to an individual in need thereof. The skilled artisan is well aware of suitable ways of administration.

Moreover, the present invention provides a method for the treatment of cancer or tumors in an individual comprising the step of administering an effective amount of an inhibitor of CRP, SAA and/or CXCL1 or a pharmaceutically acceptable salt thereof for inactivating, deleting or otherwise suppressing MDSC.

The following examples are intended to illustrate the invention further, however, they are not meant to limit the scope of the invention in a new way.

### EXAMPLES

### MATERIAL AND METHODS

**Mice.** Male *gp130f*/*f, gp130Δhepa, gp130StatΔhepa* and *gp130RasΔhepa* mice at the age of 8-12 weeks, as described previously (Klein et al., 2005) have been used. All experiments were approved by the institutional Animal Care and Use Committee. All experiments were carried out in agreement with the 'Guide for the Care and Use of Laboratory Animals' (NIH publication 86-23, revised 1985).

**Cecal ligation and puncture (CLP).** Polymicrobial sepsis was induced by CLP. Briefly, mice were anesthetized; the cecum was ligated and punctured twice with a 20G needle. Mice were monitored every 6 h for 24 h then every 12 h till the end of the experiment (between 5 and 10 days).

**LPS-induced endotoxic shock.** Mice received 20mg/kg LPS intraperitoneally and were monitored every 6 hours for 24 h then every 12 h till the end of the experiment (between 3 and 5 days).

**Cell isolation and culture.** MDSC were isolated from spleens of *gp130f*/*f* mice 5 days after CLP. After sacrifice, spleens were explanted and splenocytes were isolated by collagenase digestion. Red blood cells were lysed using Pharmlyse lysing buffer (BD Biosciences). MDSC were purified from cell suspension by magnetic cell sorting (MACS, Miltenyi Biotec) using anti-CD11b conjugated magnetic beads (Miltenyi Biotec). Purity was determined by flow cytometry. Only cell preparations with a purity of >90% for further experiments were used. Alternatively, mice were injected with 5mg/kg LPS i.p. and 5 days later CD11b⁺Gr1⁺ splenocytes were purified by FACS sorting (Influx cell sorter, BD Biosciences). For overnight culture experiments cells were kept in RPMI 1640 (Invitrogen) supplemented with 10% heat inactivated fetal calf serum (Sigma). MDSC were cultured with the following substances as indicated, murine TNF alpha (Peprotech), LPS (Sigma), SAA (Peprotech).

For generation of peritoneal macrophages mice were injected with of 1ml thioglycollate (BD Biosciences) intraperitoneally. Total cells were harvested after 72 h and plated at 1x10⁶/ml in RPMI 1640 (Invitrogen) supplemented with 10% fetal calf serum (Sigma) and 1% penicillin/streptomycin (Invitrogen). After overnight culture non-adherent cells were removed and purity of macrophages was confirmed by F4/80 staining and flow cytometry.

Primary hepatocytes were isolated from *gp130f*/*f* and *gp130Δhepa* mice by collagenase perfusion as described previously (Klein et al., 2005), 1x10⁶ cells were cultured on 6-cm culture plates with DMEM (Invitrogen) containing 4.5 g/Lglucose, 10% FCS, and penicillin/streptomycin overnight. Cells were stimulated with 50 ng/ml leukemia inhibitory factor LIF (Peprotech) for 6 h, and supernatants were harvested and stored at -80°C.

**In vitro culture of MDSC.** Culture conditions have been used previously (Delano et al., 2007). After MACS purification of splenic MDSC from control *gp130f*/*f* mice five days after CLP. Cells were plated at 10⁶ cells/ml in RPMI 1640 supplemented with 10% fetal calf serum, 2 mM I-glutamine, 200 U/ml penicillin, and 50µg/ml streptomycin, and cultured overnight with the indicated compounds. For in vitro suppression assays, purified MDSC were co-cultured with peritoneal or bone marrow derived macrophages, stimulated with LPS (Sigma) and subsequent cytokine release was measured by ELISA. In some cases MDSC were separated from macrophages in transwells (pore size 0.4 µm, Corning Inc.)

For in vitro migration studies, splenic or bone marrow derived CD11b⁺Gr1⁺ cells were seeded in transwells (pore size 3µm, Corning Inc.) and increasing amounts of recombinant KC (Peprotech) were added to the lower well. After 3h migrated cells were counted in a hemocytometer and the migration index of migrated/non-migrated cells wascalculated.

**Adoptive MDSC transfer.** MDSC were isolated as described above, *gp130Δhepa* mice received 5x10⁶ MDSC intravenously 1 h and 24 h after CLP. Control mice were treated with equal volumes of NaCl.

**Depletion of Gr1⁺ cells.** Monoclonal antibodies against Gr1 from RB6-8C5 hybridoma cells (Tepper, R.I., et al, 1992, Science 257, 548-551) have been used and *gp130f*/*f* mice received 10 mg/kg of antibodies 24 h before and 24 h after CLP. Efficiency of depletion was monitored by FACS. Control mice received non-specific isotype IgG antibodies.

**Treatment of animals with recombinant SAA, KC and IL1Ra.** Mice received 0.8 mg/kg recombinant SAA (Peprotech) or 40µg/kg KC (Peprotech) or a combination of both intravenously 1 h and 24 h after CLP. Control mice received the same volume of saline. IL-1Ra (Anakinra) (Kineret, Amgen) was injected subcutaneously every 4 h over a period of 24 h.

**Systemic neutralization of SAA.** Mice received 50mg/kg of a combination of anti-SAA monoclonal antibodies **(mc29** and **mc4, 1:**1) directed against the highly conserved (invariant) region of SAA (see above) i.p. 12h before and 33mg/kg 12h after CLP. Control mice received equivalent amounts of inactive control antibodies **(mc1).** The three antibodies are described in Linke et. al., J. Histochem, Cytochem. 32. 322328 (1984).

**Cytokine measurements.** Serum samples were collected by bleeding at the indicated time points. IL-6, TNF, IFN-y and KC were measured by ELISA (R&D systems). SAA was measured by ELISA (Biosource). IL-6, TNF, IL-12 and IL-10 were measured from cell culture supernatants by ELISA (R&D systems).

**Flow cytometry.** Cells suspensions were incubated with the flourochrome conjugated antibodies listed below and analyzed with a BD FACS Canto II (BD Biosystems). CD45, Gr1 (both BD Biosciences), CD11b (eBioscience).

**Gene-microarray analysis and real time PCR.** *Gp130f*/*f* and *gp130Δhepa* mice were sacrificed without prior treatment or 12 h after CLP. Livers were explanted and tissue samples were frozen immediately. Liver RNA samples from 3 mice per experimental group were used for microarray analysis. RNA samples were prepared and hybridized on Affymetrix GeneChip Mouse Genome 430 2.0 arrays. Gene expression levels were calculated applying the GC content-corrected robust multichip average algorithm (GCRMA) and a remapped Gene Chip Description File (CDF). Differentially expressed genes were identified using Limma statistics. Genes with a corrected p-value similar or below 0.05 and/or fold change above 1.5 or below -1.5 were considered significantly regulated. Detailed descriptions of the applied methods are available on request. Ingenuity Pathway Analysis (v5.5, Ingenuity Systems) and ErmineJ Gene Score Resampling were used to identify the most significantly changed canonical signaling pathways or Gene Ontology (GO) biological processes in *gp130f*/*f* and *gp130Δhepa* mice 12 h after sepsis onset, using a p-value similar to or below 0.05 as input criteria. Fold changes of selected genes were visualized in a heat map diagram.

Real time PCR analysis was performed in order to confirm data from the gene array. RNA was isolated using RNeasy columns (Qiagen) following the manufacturer's instructions. First strand synthesis was performed with Oligo dT primers and reverse transcription with M-MLV Reverse Transcriptase (Invitrogen). Quantitative Real Time PCR was performed using SYBR Green Reagent (Invitrogen Corp.) in ABI-Prism 7300 Real Time PCR system (Applied Bioscience). Reactions were performed in duplicate and GAPDH values were used to normalize gene expression.

**Determination of bacterial burden.** Mice were sacrificed 24 h after CLP. The peritoneal cavity was lavaged and the lavage fluid was plated on blood-agar plates and McKonkey-agar plates in serial dilutions. Spleens were removed, homogenized and plated in serial dilutions on agar plates. Colony forming units were counted after overnight incubation.

**Determination of apoptosis by Caspase-3 kinase activity.** Purified MDSC were cultured overnight with the indicated compounds. Then cells were washed and lysed at 4°C in 5 mmol/L Tris/HCl, pH 8.0, 20 µmol ethylenediaminetetraacetic acid, 0.5% Triton X-100. Lysates were clarified by centrifugation at 13,000g for 10 min. Reaction mixture contained 30 µg cellular lysates, 1000 µl assay buffer (20 mmol/L HEPES, pH 7.5, 10% glycerol, 2 mM dithiothreitol), and 20 mM caspase-3 substrate (Ac-DEVD-AMC) (BD Pharmingen). After 2 h incubation in the dark, enzymatic activity was measured in a Luminiscence Spectophotometer (LS-50; Perkin Elmer).

**Determination of apoptosis by annexin-V staining.** Cells were stained for CD45, Gr1 and CD11b, followed by incubation with annexin-V - FITC (BD Biosciences) and analyzed by flow cytometry.

**Statistical analysis.** Statistical significance was determined by unpaired two-tailed student's t-test implemented in Excel (version 11.0, Microsoft)

Herein it is shown that hepatic gp130-deficiency severely decreased survival of *gp130Δhepa* mice during polymicrobial sepsis compared to control littermates (Fig. 1A). Control mice carried loxP sites flanking Exon 16 of the gp130 gene, but did not express Cre-recombinase *(gp130f*/*f).* The protective effects of hepatic gp130 signaling required Stat3 activation, demonstrated by a markedly decreased survival of *gp130StatΔhepa* compared to *gp130f*/*f* and *gp130RasΔhepa* animals. Proinflammatory cytokines like IL-6, TNF and interferon gamma (IFN-y) are heavily involved in the pathogenesis of septic shock and subsequent organ failure. In the mouse model described herein, serum levels of these cytokines peaked at 6 h in all animals. In contrast, while the systemic cytokine levels quickly normalized in *gp130f*/*f* control and *gp130RasΔhepa* mice, they remained persistently elevated after 24 and 48 h in *gp130Δhepa* and *gp130StatΔhepa* animals.

Then, the possibility of defective bacterial clearance in mice lacking hepatic gp130/Stat3 signaling leading to elevated cytokine levels was explored. However, when examining bacterial counts of the peritoneal cavity and the spleen, by plating serial dilutions of peritoneal lavage fluid or spleen homogenates 12h after CLP, no differences between the groups have been found and nearly all animals had cleared the bacteria from the spleens after 36h. Furthermore, leukocyte recruitment to the peritoneal cavity was equal in all groups (Fig. 1 B) indicating that the innate immune response was intact. This suggested that impaired counter regulation of the initial inflammatory response rather than defective anti-bacterial defense was responsible for the exacerbated inflammation and increased mortality of *gp130Δhepa* mice. To further test this hypothesis sterile endotoxic shock by injection of lipopolysaccharide (LPS) was induced. Similar to the infection model, hepatic gp130-deficiency strongly increased mortality (Fig. 1C) and caused prolonged inflammation (Fig. 1D). Further liver-intrinsic effects of gp130-deficiency during sepsis has been studied and it has been found that *gp130Δhepa* mice showed markedly elevated liver enzyme serum levels (Fig. 1E) and pronounced hepatocyte necrosis compared to *gp130f*/*f* mice following CLP. Enhanced liver injury not only reflected the severity of the systemic inflammation but also confirmed the auto-protective function of IL-6/gp130 signaling in hepatocytes. Collectively, these experiments suggested that liver derived factors were essential to control the inflammatory response to infection.

Next it was investigated whether dysregulated inflammation in *gp130Δhepa* mice was associated with changes in the composition of immune cells in the peripheral blood, lymphoid organs and the liver. Especially it was focused on MDSCs, an immature population of myeloid (precursor) cells characterized by surface expression of CD11b and Gr1 in mice. MDSCs have recently gained significant attention for their role in malignancies. Interestingly, one previous study reported expansion of MDSCs in the bone marrow and accumulation in the spleen of septic mice (Delano et al., 2007). Therefore the expansion of CD11b⁺Gr1⁺ cells in the bone marrow, as well as their mobilization to the peripheral blood and accumulation in the spleen, lymph nodes and the liver of mice at various time points after induction of polymicrobial sepsis have been monitored. As shown herein although CD11b⁺Gr1⁺ cells expanded similarly in the bone marrow of all mice, the population of circulating CD11b⁺Gr1⁺ cells in the blood was significantly reduced in *gp130Δhepa* mice (Fig. 2A, B). Further accumulation of MDSCs in the spleens of septic *gp130f*/*f* control mice as previously reported (Delano et al., 2007) (Fig. 2A, B) has been observed. However, splenic enrichment of MDSCs after CLP was completely blocked in *gp130Δhepa* animals, as demonstrated by flow cytometry and immunofluorescence microscopy (Fig. 2A, B). Hepatic accumulation of CD11b⁺Gr1⁺ cells was also significantly decreased in *gp130Δhepa* mice. No increase of MDSCs in mesenteric lymph nodes was observed in any of the animals. These data strongly suggested that gp130-dependent signals in the liver facilitated mobilization and peripheral accumulation of MDSCs during polymicrobial sepsis.

Positive CD11b/Gr1 staining is not unique to MDSCs especially in compartments with relatively high numbers of myeloid cells like the bone marrow and the peripheral blood. The population of CD11b⁺Gr1⁺ cells is therefore not necessarily equivalent to MDSCs. In the absence of exclusive surface markers MDSCs are best defined functionally, through their ability to suppress immune responses. Therefore, their functions during sepsis were analyzed. Myeloid cells with anti-inflammatory functions have been recognized in tumor-bearing mice for a long time (Young et al., 1987), but their functional significance for tumor immune evasion, angiogenesis and metastasis has only recently been more broadly appreciated (Nagaraj et al., 2007; Yang et al., 2008). MDSCs have also been implicated in other pathological conditions like autoimmunity (Haile et al., 2008; Zhu et al., 2007). However, despite significant advances in cancer, there is only limited data on MDSC functions in infections and sepsis. Expansion of MDSCs with T cell suppressive properties during sepsis in mice has been reported (Delano et al., 2007). Unlike for many cancers, their contribution to disease progression and host survival during infections is still undefined. Thus, a goal of the present invention is to establish a link between hepatic gp130 deficiency, subsequent impairment of MDSC accumulation and reduced survival. Two independent approaches to test the relevance of MDSCs for host survival have been chosen. First Gr1⁺ MDSCs were depleted in control mice using a monoclonal antibody directed against Gr1. Depletion of MDSCs significantly increased mortality compared to mice treated with a control isotype antibody(Fig. 3A, B). Since anti-Gr1 treatment is not specific for MDSCs and would also target other Gr1⁺ cells like neutrophils, it was decided to adoptively transfer MDSCs, isolated from spleens of *gp130f*/*f* donors 5 days after CLP, into *gp130Δhepa* animals. Purified MDSCs from spleens of *gp130f*/*f* mice showed a characteristic morphological heterogeneity and immature phenotype. Around50% of CD11b⁺Gr1⁺ cells expressed Ly6G, a marker associated with the PMN subset of MDSCs. Importantly, MDSCs efficiently reversed the detrimental effects of hepatic gp130-deficiency during infection (Fig. 3C). MDSC transfer increased survival of *gp130Δhepa* mice to wildtype levels (Fig. 3C), demonstrating a key role of MDSCs in regulating inflammatory responses to infection. Previous studies had shown suppressive functions of MDSCs on T cells (Delano et al., 2007). Herein it is shown that purified sepsis-induced splenic MDSCs inhibited LPS-induced pro-inflammatory cytokine release while strongly inducing the production of IL-10 in primary macrophages (Fig. 3D, E).

This skewing effect was dependent on activation of the MDSCs *in vivo.* CD11b⁺Gr1⁺ cells that were sorted from the spleens of untreated mice were strongly impaired in their ability to inhibit IL-12 release and induce IL-10 production in LPS treated macrophages (Fig. 3E). Pre-activation of CD11b⁺Gr1⁺ cells from naïve mice with LPS *in vitro* prior to co-culture with macrophages, partly restored IL-10 induction but failed to recover inhibition of IL-12 production (Fig. 3E). Thus, MDSCs generated during infection show a strong anti-inflammatory phenotype, in contrast to splenic CD11b⁺Gr1⁺ cells under steady state conditions. The anti-inflammatory functions of MDSCs were largely dependent on direct cell-cell contact. Separation of MDSCs and macrophages in a transwell system strongly reduced IL-10 induction, however it only partially blocked IL-12 suppression, indicating that additional soluble factors like TGF-β may play a role as well. The results shown herein suggest that direct interaction of MDSCs with innate effector cells may constitute a novel auto-regulatory mechanism of innate immune responses to infection.

Hitherto, knowledge about factors and conditions that induce MDSC production and function as well as their homing to peripheral tissues or lymphoid organs is incomplete. Observations made in murine cancer models have suggested that tumor derived factors induce the expansion of MDSCs and inhibit their differentiation into DCs by activation of Janus-Kinase (JAK)2/Stat3 pathways in MDSCs (Nefedova et al., 2004). In the present model, JAK2/Stat3 signaling in hematopoetic cells was intact and no differences in the expansion of CD11b⁺Gr1⁺ cells in the bone marrow (Fig. 2A, B) has been observed. Instead, hepatocyte specific deletion of gp130/Stat3 signaling abrogated the peripheral accumulation of MDSCs. Therefore, it was aimed to identify liver-derived gp130-dependent factors that were involved in mobilization and peripheral accumulation of MDSCs, thereby controlling inflammation. Transcriptional analysis of 16,475 genes by gene-microarray in liver tissue samples taken from untreated and septic *gp130f*/*f* and *gp130Δhepa* mice revealed a total of 3,936 genes that were regulated more than 1.5 fold 12h after CLP, 2,266 of which were differentially regulated by gp130. Focusing on secreted proteins, three groups of genes with a particularly strong gp130-dependent induction were noticed: 1. Classical APPs like alpha-2-macroglobulin, CRP or serum amyloid A (SAA), 2. chemokines with chemotactic activity on myeloid cells, like CXCL1 (KC), CXCL2 or Ccl3 (MIP-1α), and 3. anti-inflammatory cytokines like IL1-receptor antagonist (IL1Ra). These results were further confirmed by real time RT PCR (Fig. 4A). Accordingly, the serum levels of SAA and CXCL1 were strongly elevated in *gp130f*/*f* but not in *gp130Δhepa* mice 24 h after CLP, showing that hepatic gp130 signaling controls the systemic levels of these proteins (Fig. 4B). Transcript levels of IL-6 were also significantly reduced in *gp 130Δhepa* mice compared to controls (8.3 *vs.* 1.9 fold induction, data not shown), which is noteworthy since it is widely believed that the liver is a major source of IL-6 and TNF during sepsis. In contrast it was found that *gp130Δhepa* mice showed elevated serum levels of IL-6 following CLP despite reduced hepatic IL-6 production. Hepatic TNF transcript levels were not found to be differentially regulated by gp130. This suggests a mainly extrahepatic production during sepsis, for example in macrophages activated by microbial products.

Having identified hepatic genes regulated by gp130 during polymicrobial infection, it was aimed to determine their functional contributions to the regulation of the inflammatory response and MDSC accumulation. To this end *gp130Δhepa* mice that failed to induce SAA, CXCL1 and IL1Ra have been treated with the respective recombinant proteins. Administration of SAA or CXCL1 alone partly restored survival (Fig. 5A, B), while a combination of both proteins completely rescued *gp130Δhepa* mice normalizing survival rates and systemic cytokine levels (Fig. 5A,B,D). In sharp contrast, treatment with the anti-inflammatory cytokine IL1Ra (Anakinra) resulted in 100% mortality of *gp130Δhepa* mice after 4d (Fig. 5C). Detrimental effects of IL-1Ra treatment may be due to impaired bacterial clearance or defective tissue repair in the absence of IL-1.

Notably, administration of recombinant KC, known as a potent chemoattractant for polymorphonuclear cells, significantly elevated the numberof circulating CD11b⁺Gr1⁺ cells in septic *gp130Δhepa* mice. Recombinant KC also induced chemotaxis of purified MDSCs in vitro. In contrast, splenic MDSC numbers were only marginally elevated in KC-treated *gp130Δhepa* animals. Thus, it was conceivable that KC was involved in mobilization of myeloid cells from the bone marrow, while accumulation of MDSCs in the spleen required additional factors. Therefore the ability of SAA to restore splenic MDSCs was tested. SAA is mostly regarded as a pro-inflammatory protein with activating properties on innate immune cells, and has also been implicated in the pathogenesis of chronic inflammation like atherosclerosis. SAA treatment of *gp130Δhepa* mice during sepsis efficiently reduced mortality (Fig. 5B, D) and inflammation but importantly, it nearly restored MDSC numbers in the spleen especially when given in combination with KC, demonstrating that SAA was sufficient to rescue *gp130Δhepa* mice during sepsis. However, it was not clear if SAA was required in the context of an intact APR. Blocking SAA activity in *gp130f*/*f* mice by injection of neutralizing anti-SAA antibodies strongly enhanced sepsis-related mortality and systemic cytokine levels (Fig. 5F, G). Given the large quantities and vast variety of APPs produced during sepsis, it was surprising to find that inhibition of SAA alone had such dramatic effects and it indicates a central role for this APP in the regulation of the inflammatory response to infection. Thus, hepatic APPs promote MDSC accumulation and regulate inflammation during sepsis. However, how MDSCs home to the spleen and not to other lymphoid organs like the mesenteric lymph node is not clear to this point and needs further investigation.

The liver might be a potent source of MDSC inducing proteins not only in sepsis but potentially also in cancer. In this respect it is also noteworthy that MDSCs have been shown to accumulate in human hepatocellular carcinoma, a cancer known to notoriously escape anti-tumor immune responses (Hoechst et al., 2008).

In addition to JAK2/Stat3, the role of which for MDSC induction has been studied mainly in malignancies, it was previously shown that the expansion of MDSCs during polymicrobial sepsis required MyD88 signaling (Delano et al., 2007). However, it is not clear if direct MyD88 activation in MDSCs, or alternatively indirect mechanisms like MyD88-dependent production of cytokines like IL-6 are responsible for these effects. The present invention does not directly address this issue, but the results demonstrate that MyD88/NFκB dependent IL-6 cytokines and subsequent gp130 signaling in hepatocytes is a crucial element for peripheral accumulation of MDSCs in sepsis. This concept is intriguing because it implicates a novel negative feedback mechanism of TLR-mediated inflammatory responses. Contraction of immune cells, particularly lymphocytes, during severe infections is a well-known phenomenon. Here, it was found a higher percentage of AnnexinV⁺ apoptotic MDSCs in spleens of *gp130Δhepa* mice compared to *gp130f*/*f* mice. Interestingly, the apoptotic fraction was significantly reduced in SAA treated *130Δhepa* animals. Thus it was reasoned that SAA promotes survival of peripheral. Therefore the potential of recombinant SAA to inhibit apoptosis of MDSCs isolated from spleens of septic mice was tested. Indeed, it was found that both recombinant SAA and supernatant of stimulated primary hepatocytes from *gp130f*/*f* but not from *gp130Δhepa* mice prevented TNF-induced MDSC apoptosis (Fig. 6). Primary hepatocytes isolated from control *gp130f*/*f* and *gp130Δhepa* mice were stimulated with leukemia inhibitory factor, a strong gp130 agonist, to induce production of gp130 dependent proteins. Addition of SAA to the supernatant of *gp130Δhepa* hepatocytes fully restored the anti-apoptotic capacity (Fig. 6), demonstrating that liver derived SAA, produced in response to gp130 activation protects MDSCs from apoptosis. Therefore, SAA inhibits MDSC apoptosis in vitro and in vivo. The mechanism underlying the anti-apoptotic effect of SAA is not clear at present. Interestingly, SAA was recently identified as an endogenous TLR2 ligand activating MyD88 signaling (Cheng et al., 2008a). It is therefore conceivable that SAA activates TLR2/MyD88 in MDSCs to protect them from apoptosis. This would also be in line with the previously reported requirement for MyD88 for MDSC expansion during sepsis (Delano et al., 2007).

Collectively, these results define a pivotal role of liver derived proteins in regulating MDSC mobilization and survival in sepsis, and the production of these factors requires gp130/Stat3 signaling in hepatocytes.

In summary, the present invention demonstrates that MDSCs have potent host protective anti-inflammatory functions during polymicrobial infections. *Gp130Δhepa* animals that failed to accumulate MDSCs in the spleen suffered from dysregulated inflammation and increased mortality. Adoptive transfer of splenic MDSCs into *gp130Δhepa* mice conferred protection. It is known that adaptive immune cells are required to control innate immune responses to infections (Kim et al., 2007). As shown here (Fig. 3) and previously in cancer-derived MDSCs (Sinha et al., 2007), MDSCs can directly interact with macrophages and inhibit proinflammatory cytokine release. Thus, they represent an auto-regulatory component of the innate immune system, contributing to carefully balance the inflammatory response to bacterial infection and sepsis.

The results as shown herein directly link MDSC functions during infection to hepatic APPs induced by gp130/Stat3 activation. Our study shows that APPs are crucial regulators of inflammatory responses to infection and highlights the close relationship between hepatocytes and innate immune cells and SAA plays a key role in this regulatory process.
Delano, M.J., et al., 2007. J Exp Med. 204:1463-74.
Haile, L.A., et al., 2008. 135:871-81, 881 e1-5.
Hoechst, B., et al., 2008. Gastroenterology. 135:234-43.
Kim, K.D., et al., 2007. Nat Med. 13:1248-52.
Klein, C., T. et al., 2005. J Clin Invest. 115:860-9.
Nagaraj, S., et al., 2007. Nat Med. 13:828-35.
Nefedova, et al., 2004. J Immunol. 172:464-74.
Rebouissou, S., et al., 2009. Nature. 457:200-4.
Sinha, P., et al., 2007. J Immunol. 179:9
Yang, L., et al., 2008. Cancer Cell. 13:23-35.
Young, et al., 1987. Cancer Res. 47:100-5.
Zhu, B., et al., 2007. J Immunol. 179:5228-37.

## Claims

1. Pharmaceutical composition containing as an active ingredient an effective amount of inhibitors of SAA, CRP and/or CXCL-1 activity, or a pharmaceutically acceptable salt for the use in the treatment of dysregulated inflammatory diseases or conditions and/or malignancies and/or cancer and/or tumors by influencing the activity of myeloid-derived suppressor cells (MDSC).

2. Pharmaceutical composition according to claim 1 for use in the treatment of autoinflammatory diseases or autoinflammatory diseases with autoimmune components or autoimmune diseases including IBD, rheumatoid arthritis, psoriasis, multiple sclerosis, autoimmune hepatitis, transplant rejection, graft vs. host and host vs. graft disease, acute toxic and chronic toxic liver damage, local and systemic inflammatory diseases.

3. Pharmaceutical composition according to claim 1 or 2 for use in the therapy of a systemic inflammatory response syndrome, sepsis, severe sepsis, septic shock, multi organ dysfunction syndrome, multiple organ failure syndrome or acute transplant rejection.

4. Pharmaceutical composition according to any one of the preceding claims containing as an active ingredient an effective amount of an inhibitor of SAA, CRP and/or CXCL-1 activity, in particular neutralising antibodies against SAA, CRP and/or CXCL-1, or a pharmaceutically acceptable salt thereof for use in the treatment of malignancies or cancer and/or tumors or dysregulated inflammatory disease or conditions whereby the inflammation response is suppressed, through inhibition of MDSC accumulation, frequency and/or activity.

5. Pharmaceutical composition according to any one of the preceding claims wherein the active ingredient is an inhibitor of SAA, CRP and/or CXCL-1 activity or a pharmaceutically acceptable salt thereof.

6. Pharmaceutical composition according to any one of the preceding claims further comprising pharmaceutical additives or auxiliary substances and, preferably, a pharmaceutically acceptable carrier, excipient or diluent.

7. Use of an effective amount of inhibitors of SAA, CRP and/or CXCL-1 activity or a pharmaceutically acceptable salt thereof for reducing the frequency, accumulation and/or activity, and/or for depleting MDSC for treating diseases or conditions **characterised by** suppressed immune response.

8. Method for the treatment of malignancies and/or cancer and/or tumors in individuals by administering an effective amount of an inhibitor of SAA, CRP and/or CXCL-1 or a pharmaceutically acceptable salt thereof for inactivating, depleting or otherwise suppressing MDSC.


SEQUENCE LISTING

<110>  RWTH Aachen

<120>  Pharmaceutical composition for acute phase targeted treatment of
       acute and chronic dysregulated inflammatory diseases or
       conditions

<130>  4589-003

<160>  10

<170>  PatentIn version 3.3

<210>  1
<211>  5
<212>  PRT
<213>  artificial

<220>
<223>  peptide

<400>  1

Tyr Ile Gly Ser Asp
1               5


<210>  2
<211>  5
<212>  PRT
<213>  artificial

<220>
<223>  peptide

<400>  2

Tyr Ile Gly Ser Arg
1               5


<210>  3
<211>  1103
<212>  DNA
<213>  human CXCL1


<220>
<221>  CDS
<222>  (80)..(403)

<400>  3
cacagagccc gggccgcagg cacctcctcg ccagctcttc cgctcctctc acagccgcca      60

gacccgcctg ctgagcccc atg gcc cgc gct gct ctc tcc gcc gcc ccc agc      112
                     Met Ala Arg Ala Ala Leu Ser Ala Ala Pro Ser
                     1               5                   10

aat ccc cgg ctc ctg cga gtg gca ctg ctg ctc ctg ctc ctg gta gcc      160
Asn Pro Arg Leu Leu Arg Val Ala Leu Leu Leu Leu Leu Leu Val Ala
            15                  20                  25

gct ggc cgg cgc gca gca gga gcg tcc gtg gcc act gaa ctg cgc tgc     208
Ala Gly Arg Arg Ala Ala Gly Ala Ser Val Ala Thr Glu Leu Arg Cys
        30                  35                  40

cag tgc ttg cag acc ctg cag gga att cac ccc aag aac atc caa agt     256
Gln Cys Leu Gln Thr Leu Gln Gly Ile His Pro Lys Asn Ile Gln Ser
    45                  50                  55

gtg aac gtg aag tcc ccc gga ccc cac tgc gcc caa acc gaa gtc ata     304
Val Asn Val Lys Ser Pro Gly Pro His Cys Ala Gln Thr Glu Val Ile
60                  65                  70                  75

gcc aca ctc aag aat ggg cgg aaa gct tgc ctc aat cct gca tcc ccc     352
Ala Thr Leu Lys Asn Gly Arg Lys Ala Cys Leu Asn Pro Ala Ser Pro
                80                  85                  90

ata gtt aag aaa atc atc gaa aag atg ctg aac agt gac aaa tcc aac     400
Ile Val Lys Lys Ile Ile Glu Lys Met Leu Asn Ser Asp Lys Ser Asn
            95                  100                 105

tga ccagaaggga ggaggaagct cactggtggc tgttcctgaa ggaggccctg     453

cccttatagg aacagaagag gaaagagaga cacagctgca gaggccacct ggattgtgcc     513

taatgtgttt gagcatcgct taggagaagt cttctattta tttatttatt cattagtttt     573

gaagattcta tgttaatatt ttaggtgtaa aataattaag ggtatgatta actctacctg     633

cacactgtcc tattatattc attctttttg aaatgtcaac cccaagttag ttcaatctgg     693

attcatattt aatttgaagg tagaatgttt tcaaatgttc tccagtcatt atgttaatat     753

ttctgaggag cctgcaacat gccagccact gtgatagagg ctggcggatc caagcaaatg     813

gccaatgaga tcattgtgaa ggcaggggaa tgtatgtgca catctgtttt gtaactgttt     873

agatgaatgt cagttgttat ttattgaaat gatttcacag tgtgtggtca acatttctca     933

tgttgaaact ttaagaacta aaatgttcta aatatccctt ggacatttta tgtctttctt     993

gtaaggcata ctgccttgtt taatggtagt tttacagtgt ttctggctta gaacaaaggg     1053

gcttaattat tgatgttttc atagagaata taaaaataaa gcacttatag     1103

<210>  4
<211>  107
<212>  PRT
<213>  human CXCL1

<400>  4

Met Ala Arg Ala Ala Leu Ser Ala Ala Pro Ser Asn Pro Arg Leu Leu
1               5                   10                  15

Arg Val Ala Leu Leu Leu Leu Leu Leu Val Ala Ala Gly Arg Arg Ala
            20                  25                  30

Ala Gly Ala Ser Val Ala Thr Glu Leu Arg Cys Gln Cys Leu Gln Thr
        35                  40                  45

Leu Gln Gly Ile His Pro Lys Asn Ile Gln Ser Val Asn Val Lys Ser
    50                  55                  60

Pro Gly Pro His Cys Ala Gln Thr Glu Val Ile Ala Thr Leu Lys Asn
65                  70                  75                  80

Gly Arg Lys Ala Cys Leu Asn Pro Ala Ser Pro Ile Val Lys Lys Ile
                85                  90                  95

Ile Glu Lys Met Leu Asn Ser Asp Lys Ser Asn
            100                 105

<210>  5
<211>  716
<212>  DNA
<213>  human SAA1

<220>
<221>  CDS
<222>  (224)..(592)

<400>  5
aggctcagta taaatagcag ccaccgctcc ctggcaggca gggacccgca gctcagctac      60

agcacagatc aggtgaggag cacaccaagg agtgattttt aaaacttact ctgttttctc     120

tttcccaaca agattatcat ttcctttaaa aaaaatagtt atcctggggc atacagccat     180

accattctga aggtgtctta tctcctctga tctagagagc acc atg aag ctt ctc       235
                                                Met Lys Leu Leu
                                                1

acg ggc ctg gtt ttc tgc tcc ttg gtc ctg ggt gtc agc agc cga agc       283
Thr Gly Leu Val Phe Cys Ser Leu Val Leu Gly Val Ser Ser Arg Ser
5                   10                  15                  20

ttc ttt tcg ttc ctt ggc gag gct ttt gat ggg gct cgg gac atg tgg       331
Phe Phe Ser Phe Leu Gly Glu Ala Phe Asp Gly Ala Arg Asp Met Trp
                25                  30                  35

aga gcc tac tct gac atg aga gaa gcc aat tac atc ggc tca gac aaa       379
Arg Ala Tyr Ser Asp Met Arg Glu Ala Asn Tyr Ile Gly Ser Asp Lys
            40                  45                  50

tac ttc cat gct cgg ggg aac tat gat gct gcc aaa agg gga cct ggg       427
Tyr Phe His Ala Arg Gly Asn Tyr Asp Ala Ala Lys Arg Gly Pro Gly
        55                  60                  65

ggt gcc tgg gct gca gaa gtg atc agc gat gcc aga gag aat atc cag       475
Gly Ala Trp Ala Ala Glu Val Ile Ser Asp Ala Arg Glu Asn Ile Gln
    70                  75                  80

aga ttc ttt ggc cat ggt gcg gag gac tcg ctg gct gat cag gct gcc       523
Arg Phe Phe Gly His Gly Ala Glu Asp Ser Leu Ala Asp Gln Ala Ala
85                  90                  95                  100

aat gaa tgg ggc agg agt ggc aaa gac ccc aat cac ttc cga cct gct       571

Asn Glu Trp Gly Arg Ser Gly Lys Asp Pro Asn His Phe Arg Pro Ala
                105                 110                 115

ggc ctg cct gag aaa tac tga gcttcctctt cactctgctc tcaggagatc      622
Gly Leu Pro Glu Lys Tyr
            120

tggctgtgag gccctcaggg cagggataca aagcggggag agggtacaca atgggtatct    682

aataaatact taagaggtgg aatttgtgga aact                                716


<210>  6
<211>  122
<212>  PRT
<213>  human SAA1

<400>  6

Met Lys Leu Leu Thr Gly Leu Val Phe Cys Ser Leu Val Leu Gly Val
1               5                   10                  15


Ser Ser Arg Ser Phe Phe Ser Phe Leu Gly Glu Ala Phe Asp Gly Ala
            20                  25                  30


Arg Asp Met Trp Arg Ala Tyr Ser Asp Met Arg Glu Ala Asn Tyr Ile
        35                  40                  45


Gly Ser Asp Lys Tyr Phe His Ala Arg Gly Asn Tyr Asp Ala Ala Lys
    50                  55                  60


Arg Gly Pro Gly Gly Ala Trp Ala Ala Glu Val Ile Ser Asp Ala Arg
65                  70                  75                  80


Glu Asn Ile Gln Arg Phe Phe Gly His Gly Ala Glu Asp Ser Leu Ala
                85                  90                  95


Asp Gln Ala Ala Asn Glu Trp Gly Arg Ser Gly Lys Asp Pro Asn His
            100                 105                 110


Phe Arg Pro Ala Gly Leu Pro Glu Lys Tyr
        115                 120


<210>  7
<211>  545
<212>  DNA
<213>  human SAA2


<220>
<221>  CDS
<222>  (38)..(406)

<400>  7
agggacccgc agctcagcta cagcacagat cagcacc atg aag ctt ctc acg ggc      55

Met Lys Leu Leu Thr Gly
1               5

ctg gtt ttc tgc tcc ttg gtc ctg agt gtc agc agc cga agc ttc ttt     103
Leu Val Phe Cys Ser Leu Val Leu Ser Val Ser Ser Arg Ser Phe Phe
            10                  15                  20

tcg ttc ctt ggc gag gct ttt gat ggg gct cgg gac atg tgg aga gcc     151
Ser Phe Leu Gly Glu Ala Phe Asp Gly Ala Arg Asp Met Trp Arg Ala
        25                  30                  35

tac tct gac atg aga gaa gcc aat tac atc ggc tca gac aaa tac ttc     199
Tyr Ser Asp Met Arg Glu Ala Asn Tyr Ile Gly Ser Asp Lys Tyr Phe
    40                  45                  50

cat gct cgg ggg aac tat gat gct gcc aaa agg gga cct ggg ggt gcc     247
His Ala Arg Gly Asn Tyr Asp Ala Ala Lys Arg Gly Pro Gly Gly Ala
55                  60                  65                  70

tgg gct gca gaa gtg atc agc aat gcc aga gag aat atc cag aga ctc     295
Trp Ala Ala Glu Val Ile Ser Asn Ala Arg Glu Asn Ile Gln Arg Leu
                75                  80                  85

aca ggc cgt ggt gcg gag gac tcg ctg gcc gat cag gct gcc aat aaa     343
Thr Gly Arg Gly Ala Glu Asp Ser Leu Ala Asp Gln Ala Ala Asn Lys
            90                  95                  100

tgg ggc agg agt ggc aga gac ccc aat cac ttc cga cct gct ggc ctg     391
Trp Gly Arg Ser Gly Arg Asp Pro Asn His Phe Arg Pro Ala Gly Leu
        105                 110                 115

cct gag aaa tac tga gcttcctctt cactctgctc tcaggagacc tggctatgag     446
Pro Glu Lys Tyr
    120

gccctcgggg cagggataca aagttagtga ggtctatgtc cagagaagct gagatatggc     506

atataatagg catctaataa atgcttaaga ggtggaaaa                            545


<210>  8
<211>  122
<212>  PRT
<213>  human SAA2

<400>  8

Met Lys Leu Leu Thr Gly Leu Val Phe Cys Ser Leu Val Leu Ser Val
1               5                   10                  15


Ser Ser Arg Ser Phe Phe Ser Phe Leu Gly Glu Ala Phe Asp Gly Ala
            20                  25                  30


Arg Asp Met Trp Arg Ala Tyr Ser Asp Met Arg Glu Ala Asn Tyr Ile
        35                  40                  45


Gly Ser Asp Lys Tyr Phe His Ala Arg Gly Asn Tyr Asp Ala Ala Lys
    50                  55                  60

Arg Gly Pro Gly Gly Ala Trp Ala Ala Glu Val Ile Ser Asn Ala Arg
65                  70                  75                  80

Glu Asn Ile Gln Arg Leu Thr Gly Arg Gly Ala Glu Asp Ser Leu Ala
                85                  90                  95

Asp Gln Ala Ala Asn Lys Trp Gly Arg Ser Gly Arg Asp Pro Asn His
            100                 105                 110

Phe Arg Pro Ala Gly Leu Pro Glu Lys Tyr
        115                 120

<210>  9
<211>  2024
<212>  DNA
<213>  human CRP

<220>
<221>  CDS
<222>  (105)..(779)

<400>  9
aaggcaagag atctaggact tctagcccct gaactttcag ccgaatacat cttttccaaa      60

ggagtgaatt caggcccttg tatcactggc agcaggacgt gacc atg gag aag ctg      116
                                                 Met Glu Lys Leu
                                                 1

ttg tgt ttc ttg gtc ttg acc agc ctc tct cat gct ttt ggc cag aca      164
Leu Cys Phe Leu Val Leu Thr Ser Leu Ser His Ala Phe Gly Gln Thr
5                   10                  15                  20

gac atg tcg agg aag gct ttt gtg ttt ccc aaa gag tcg gat act tcc      212
Asp Met Ser Arg Lys Ala Phe Val Phe Pro Lys Glu Ser Asp Thr Ser
                25                  30                  35

tat gta tcc ctc aaa gca ccg tta acg aag cct ctc aaa gcc ttc act      260
Tyr Val Ser Leu Lys Ala Pro Leu Thr Lys Pro Leu Lys Ala Phe Thr
            40                  45                  50

gtg tgc ctc cac ttc tac acg gaa ctg tcc tcg acc cgt ggg tac agt      308
Val Cys Leu His Phe Tyr Thr Glu Leu Ser Ser Thr Arg Gly Tyr Ser
        55                  60                  65

att ttc tcg tat gcc acc aag aga caa gac aat gag att ctc ata ttt      356
Ile Phe Ser Tyr Ala Thr Lys Arg Gln Asp Asn Glu Ile Leu Ile Phe
    70                  75                  80

tgg tct aag gat ata gga tac agt ttt aca gtg ggt ggg tct gaa ata      404
Trp Ser Lys Asp Ile Gly Tyr Ser Phe Thr Val Gly Gly Ser Glu Ile
85                  90                  95                  100

tta ttc gag gtt cct gaa gtc aca gta gct cca gta cac att tgt aca      452
Leu Phe Glu Val Pro Glu Val Thr Val Ala Pro Val His Ile Cys Thr
                105                 110                 115

agc tgg gag tcc gcc tca ggg atc gtg gag ttc tgg gta gat ggg aag      500
Ser Trp Glu Ser Ala Ser Gly Ile Val Glu Phe Trp Val Asp Gly Lys

120                 125                 130

ccc agg gtg agg aag agt ctg aag aag gga tac act gtg ggg gca gaa      548
Pro Arg Val Arg Lys Ser Leu Lys Lys Gly Tyr Thr Val Gly Ala Glu
        135                 140                 145

gca agc atc atc ttg ggg cag gag cag gat tcc ttc ggt ggg aac ttt      596
Ala Ser Ile Ile Leu Gly Gln Glu Gln Asp Ser Phe Gly Gly Asn Phe
    150                 155                 160

gaa gga agc cag tcc ctg gtg gga gac att gga aat gtg aac atg tgg      644
Glu Gly Ser Gln Ser Leu Val Gly Asp Ile Gly Asn Val Asn Met Trp
165                 170                 175                 180

gac ttt gtg ctg tca cca gat gag att aac acc atc tat ctt ggc ggg      692
Asp Phe Val Leu Ser Pro Asp Glu Ile Asn Thr Ile Tyr Leu Gly Gly
                185                 190                 195

ccc ttc agt cct aat gtc ctg aac tgg cgg gca ctg aag tat gaa gtg      740
Pro Phe Ser Pro Asn Val Leu Asn Trp Arg Ala Leu Lys Tyr Glu Val
            200                 205                 210

caa ggc gaa gtg ttc acc aaa ccc cag ctg tgg ccc tga ggcccagctg      789
Gln Gly Glu Val Phe Thr Lys Pro Gln Leu Trp Pro
        215                 220

tgggtcctga aggtacctcc cggtttttta caccgcatgg gccccacgtc tctgtctctg      849

gtacctcccg cttttttaca ctgcatggtt cccacgtctc tgtctctggg cctttgttcc      909

cctatatgca ttgcaggcct gctccaccct cctcagcgcc tgagaatgga ggtaaagtgt      969

ctggtctggg agctcgttaa ctatgctggg aaacggtcca aaagaatcag aatttgaggt     1029

gttttgtttt catttttatt tcaagttgga cagatcttgg agataatttc ttacctcaca     1089

tagatgagaa aactaacacc cagaaaggag aaatgatgtt ataaaaaact cataaggcaa     1149

gagctgagaa ggaagcgctg atcttctatt taattcccca cccatgaccc ccagaaagca     1209

ggagggcatt gcccacattc acagggctct tcagtctcag aatcaggaca ctggccaggt     1269

gtctggtttg ggtccagagt gctcatcatc atgtcataga actgctgggc ccaggtctcc     1329

tgaaatggga agcccagcaa taccacgcag tccctccact ttctcaaagc acactggaaa     1389

ggccattaga attgccccag cagagcagat ctgctttttt tccagagcaa aatgaagcac     1449

taggtataaa tatgttgtta ctgccaagaa cttaaatgac tggtttttgt ttgcttgcag     1509

tgctttctta attttatggc tcttctggga aactcctccc cttttccaca cgaaccttgt     1569

ggggctgtga attctttctt catccccgca ttcccaatat acccaggcca caagagtgga     1629

cgtgaaccac agggtgtcct gtcagaggag cccatctccc atctccccag ctccctatct     1689

ggaggatagt tggatagtta cgtgttccta gcaggaccaa ctacagtctt cccaaggatt     1749

gagttatgga ctttgggagt gagacatctt cttgctgctg gatttccaag ctgagaggac     1809

gtgaacctgg gaccaccagt agccatcttg tttgccacat ggagagagac tgtgaggaca     1869

gaagccaaac tggaagtgga ggagccaagg gattgacaaa caacagagcc ttgaccacgt     1929

ggagtctctg aatcagcctt gtctggaacc agatctacac ctggactgcc caggtctata 1989

agccaataaa gcccctgttt acttgaaaaa aaaaa 2024

<210> 10
<211> 224
<212> PRT
<213> human CRP

<400> 10

Met Glu Lys Leu Leu Cys Phe Leu Val Leu Thr Ser Leu Ser His Ala
1               5                   10                  15

Phe Gly Gln Thr Asp Met Ser Arg Lys Ala Phe Val Phe Pro Lys Glu
            20                  25                  30

Ser Asp Thr Ser Tyr Val Ser Leu Lys Ala Pro Leu Thr Lys Pro Leu
        35                  40                  45

Lys Ala Phe Thr Val Cys Leu His Phe Tyr Thr Glu Leu Ser Ser Thr
    50                  55                  60

Arg Gly Tyr Ser Ile Phe Ser Tyr Ala Thr Lys Arg Gln Asp Asn Glu
65                  70                  75                  80

Ile Leu Ile Phe Trp Ser Lys Asp Ile Gly Tyr Ser Phe Thr Val Gly
                85                  90                  95

Gly Ser Glu Ile Leu Phe Glu Val Pro Glu Val Thr Val Ala Pro Val
            100                 105                 110

His Ile Cys Thr Ser Trp Glu Ser Ala Ser Gly Ile Val Glu Phe Trp
        115                 120                 125

Val Asp Gly Lys Pro Arg Val Arg Lys Ser Leu Lys Lys Gly Tyr Thr
    130                 135                 140

Val Gly Ala Glu Ala Ser Ile Ile Leu Gly Gln Glu Gln Asp Ser Phe
145                 150                 155                 160

Gly Gly Asn Phe Glu Gly Ser Gln Ser Leu Val Gly Asp Ile Gly Asn
                165                 170                 175

Val Asn Met Trp Asp Phe Val Leu Ser Pro Asp Glu Ile Asn Thr Ile
            180                 185                 190

Tyr Leu Gly Gly Pro Phe Ser Pro Asn Val Leu Asn Trp Arg Ala Leu
        195                 200                 205

Lys Tyr Glu Val Gln Gly Glu Val Phe Thr Lys Pro Gln Leu Trp Pro
    210                 215                 220